# FASCICULE DE BREVET EUROPEEN

(11) **EP 2 464 333 B1**
(45) Date de publication et mention de la délivrance du brevet: **29.06.2016**
(21) Numéro de dépôt: 10761038.8
(22) Date de dépôt: 11.08.2010
(51) Int. Cl.: A61K 9/16, A61K 31/635, A61K 9/00, A61K 9/14, A61K 31/341, A61K 31/4422, A61K 31/155, A61P 3/10, A61P 7/10, A61P 9/12, A61K 31/166, A61K 31/517, A61K 31/401, A61K 31/19, A61K 31/485, A61K 31/165, A61K 31/137, A61K 31/352

(54) **MICROGRANULÉS FLOTTANTS**
SCHWIMMENDES MIKROGRANULAT
FLOATING MICROGRANULES

(30) Priorité: 12.08.2009 FR 0955641
(43) Date de publication de la demande: 20.06.2012
(73) Titulaire: Debregeas Et Associes Pharma, 75008 Paris (FR)
(72) Inventeur: LEBON, Christophe, F-28260 Rouvres (FR); SUPLIE, Pascal, F-27400 Montaure (FR)
(74) Mandataire: Gallochat, Alain
(86) Numéro de dépôt international: PCT/FR2010/051691
(87) Numéro de publication internationale: WO 2011/018582

(56) Documents cités:
- EP-A2- 0 235 718
- WO-A1-00/66089
- WO-A1-2010/055260
- WO-A2-2005/101983
- US-A- 4 101 650
- US-A1- 2007 092 565
- US-B1- 6 214 386
- ROUGE N ET AL: "Buoyancy and drug release patterns of floating minitablets containing piretanide and atenolo as model drugs", PHARMACEUTICAL DEVELOPMENT AND TECHNOLOGY, NEW YORK, NY, US, vol. 3, no. 1, 1 janvier 1998 (1998-01-01) , pages 73-84, XP008121970, ISSN: 1083-7450
- ELKHESHEN S A ET AL: "IN VITRO AND IN VIVO EVALUATION OF FLOATING CONTROLLED RELEASE DOSAGE FORMS OF VERAPAMIL HYDROCHLORIDE", PHARMAZEUTISCHE INDUSTRIE, AULENDORF, DE, vol. 66, no. 11, 1 janvier 2004 (2004-01-01), pages 1364-1372, XP008076033, ISSN: 0031-711X
- SAWICKI W ET AL: "Compressibility of floating pellets with verapamil hydrochloride coated with dispersion Kollicoat SR 30 D", EUROPEAN JOURNAL OF PHARMACEUTICS AND BIOPHARMACEUTICS, ELSEVIER SCIENCE PUBLISHERS B.V., AMSTERDAM, NL, vol. 60, no. 1, 1 mai 2005 (2005-05-01), pages 153-158, XP025317652, ISSN: 0939-6411, DOI: DOI:10.1016/J.EJPB.2004.11.003 [extrait le 2005-05-01]
- SAUZET C ET AL: "An innovative floating gastro retentive dosage system: Formulation and in vitro evaluation", INTERNATIONAL JOURNAL OF PHARMACEUTICS, ELSEVIER BV, NL, vol. 378, no. 1-2, 13 août 2009 (2009-08-13), pages 23-29, XP026348592, ISSN: 0378-5173 [extrait le 2009-05-22]
- REDDY L H V ET AL: "Floating dosage systems in drug delivery", CRITICAL REVIEWS IN THERAPEUTIC DRUG CARRIER SYSTEMS, BEGELL HOUSE PUBLISHING INC, US, vol. 19, no. 6, 1 January 2002 (2002-01-01), pages 553-585, XP009142220, ISSN: 0743-4863, DOI: 10.1615/CRITREVTHERDRUGCARRIERSYST.V19.I6. 20

## Description

La présente invention a pour objet des microgranulés flottants, ainsi que leur procédé de préparation.

De toutes les voies d'administration, la voie orale reste la voie de choix et donc la plus utilisée dans le domaine thérapeutique.

A ce titre, la physiologie du tractus gastro-intestinal a été largement étudiée afin d'optimiser les phénomènes d'absorption et d'élimination qui régulent la pharmacocinétique d'un médicament.

Le tractus gastro-intestinal a ainsi été modélisé et étudié selon différents paramètres (transit, pH, surface, présence de récepteurs ou transporteurs spécifiques). En fonction de leurs caractéristiques physicochimiques intrinsèques, les principes actifs contenus dans les formes médicamenteuses sont absorbés à des niveaux bien précis du tube digestif.

On dispose ainsi de différentes formes orales en fonction de l'effet recherché : forme à libération prolongée, forme à libération retardée, formes bioadhésives permettant de contrôler la durée et la localisation de la libération du principe actif.

Un paramètre reste difficile à maîtriser : il s'agit de la vidange gastrique ; celle-ci est en effet soumise à une forte variabilité, ce qui est préjudiciable pour la bonne reproductibilité de l'effet thérapeutique recherché. Ce problème est majeur pour les substances médicamenteuses absorbées très haut dans le tube digestif et il s'en suit une perte de biodisponibilité.

Pour remédier à ceci, des solutions galéniques ont été apportées, visant à augmenter le temps de résidence dans l'estomac : différentes formes flottantes ont ainsi été développées.

La demande internationale WO 01/10417 décrit une composition pharmaceutique se présentant sous forme d'un comprimé flottant dans l'estomac, constituée d'une phase active comprenant un principe actif en association avec un ou plusieurs excipients et une phase non active comprenant un système générateur de gaz (CO₂) et un polymère hydrophile ou un composé minéral poreux. De préférence, ledit système générateur comprend, en mélange intime, un carbonate de métal alcalin ou alcalino-terreux ou un bicarbonate de métal alcalin, en association avec un acide choisi notamment parmi les acides mono- et polycarboxyliques. Ce mélange intime forme un couple effervescent.

La demande internationale WO 01/80822 concerne des granulés effervescents et leur procédé de préparation. Ces granulés sont constitués d'un couple effervescent et d'un liant extrudable thermofusible. Le couple effervescent est une combinaison d'un agent acide et d'un agent alcalin, ceci provoquant la formation d'un gaz en présence d'eau. Ainsi, les formulations décrites dans WO 01/80822 comprennent une combinaison agent acide / agent basique.

La demande internationale WO 02/085336 concerne des comprimés orodispersibles comprenant au moins un principe actif, un mélange d'excipients, des granules effervescents constitués d'un mélange d'un agent acide, d'un agent alcalin et d'un liant extrudable à chaud. Ces comprimés se désagrègent dans la cavité buccale au contact de la salive en moins de 60 secondes.

Les travaux de Goole (International Journal of Pharmaceutics, 334, 2007, 35-41) décrivent eux des mini-comprimés flottants, de nature matricielle ou enrobée, à libération prolongée. Ces mini-comprimés contiennent systématiquement au minimum un agent actif, un agent liant et un mélange effervescent (acide tartarique, bicarbonate de sodium et carbonate de calcium).

Sheth et Tossounian (US 4 424 235) décrivent eux une gélule flottante à libération prolongée basée sur le gonflement d'un dérivé cellulosique en présence de liquide.

Le brevet US 4101 650 (UMEZAWA HAMAO) décrit un produit actif localement dans l'estomac à action prolongée, le temps de résidence dudit produit dans l'estomac étant de 3-5 heures avec une prise après le repas ; il ne s'agit donc pas d'une forme à libération immédiate spécifiquement visée par la Demanderesse.

La demande WO 2005/101983 (SUN PHARMACEUTICAL) est relative à des formes matricielles d'une composition pharmaceutique, à savoir un comprimé ou une gélule, où le principe actif peut se situer indifféremment au coeur de la formulation, ou à sa surface où les deux, mais ne décrit ni ne suggère une formulation où le principe actif et l'agent générateur de gaz sont mélangés dans la couche superficielle entourant le coeur. Une telle structure diffère de celle de la Demanderesse.

L'article REDDY L H V ET AL « Floating dosage systems in drug delivery », CRITICAL REVIEWS IN THERAPEUTIC DRUG CARRIER SYSTEMS, BEGELL HOUSE PUBLISHING INC. vol. 19 no. 6, 1er janvier 2002 pages 553-585, décrit un système à libération prolongée et non pas une formulation à action immédiate spécifiquement visée par la Demanderesse.

La demande européenne EP 0 235 718 (EISAI CO LTD) décrit une formulation où l'élément alcalin générant l'effervescence est situé extérieurement au coeur de ladite formulation dans lequel le principe actif est présent. Une telle structure diffère de celle de la Demanderesse.

Ainsi, à ce jour, la grande majorité des compositions listées ci-dessus concerne des comprimés comprenant un mélange acide/base pour l'effervescence.

Toutefois, le choix de la forme comprimé dans le développement d'une forme flottante n'est pas optimal car il s'agit d'une forme monolithique qui est donc soumise à un phénomène de tout ou rien lors de la vidange gastrique ce qui ajoute encore à la forte variabilité.

La vidange gastrique dépend en effet de la nature, du volume, de la taille et de la digestibilité des éléments solides présents dans l'estomac. Les liquides, mais également les particules solides de diamètre inférieur à 3-5 mm passent facilement le pylore et seront donc vidangés rapidement lors de la phase digestive. Ce type de particules est donc a priori pénalisant pour les principes actifs absorbés très haut dans le tube digestif. Pour les particules de taille plus importante comme les formes monolithiques, elles sont retenues par la barrière pylorique et ne seront vidangées que dans les périodes inter-digestives. Finalement, en fonction du moment de la journée (à jeun ou période post prandiale), le transit gastrique pour ces formes monolithiques sera beaucoup plus conséquent et pourra varier de quelques dizaines de minutes à plusieurs heures.

Egalement de par sa forme et son poids intrinsèques, le comprimé s'avère problématique pour obtenir une bonne flottaison de la forme médicamenteuse. Il devient nécessaire dans ce cas d'utiliser une quantité importante d'agent effervescent pour obtenir la flottaison souhaitée.

Les systèmes susmentionnés doivent alors utiliser un couple agent acide / agent basique capable de provoquer cette effervescence ce qui ajoute encore au poids et aux dimensions du comprimé et donc finalement gène la flottaison souhaitée. Ainsi, les travaux de Goole susmentionnés consistent à développer des mini-comprimés d'un diamètre de trois millimètres mais utilisant un couple acide / base pour l'effervescence.

La proximité d'un agent acide et d'un agent basique au sein d'une même forme médicamenteuse est problématique en terme de stabilité et notamment dans des conditions d'humidité relative élevée. Cela suppose d'utiliser des conditionnements adaptés (aluminium ou PVDC, voire flacons avec déshydratant).

La présente invention a pour but d'apporter une solution à tous les inconvénients susmentionnés.

Elle a donc pour but de fournir une forme orale permettant d'améliorer la biodisponibilité et la reproductibilité de l'effet thérapeutique pour les principes actifs à fenêtre d'absorption étroite ou absorbés haut dans le tube digestif.

Un autre but de l'invention consiste à fournir une forme multiparticulaire flottante à libération prolongée, particulièrement utile pour diminuer la variabilité et donc la reproductibilité et permettant de se prémunir des inconvénients observés avec les formes monolithiques que sont les gélules ou les comprimés.

Un autre but de la présente invention consiste à fournir une forme multiparticulaire flottante préservant la paroi de l'estomac en diminuant les risques d'intolérance locale.

La présente invention consiste donc à fournir une composition spécifiquement élaborée pour obtenir une forme médicamenteuse suffisamment titrée en principe actif tout en gardant une taille suffisamment restreinte pour obtenir une flottaison satisfaisante, et plus particulièrement pour les principes actifs présentant une absorption haute dans le tractus digestif.

La présente invention concerne donc des granulés flottants comprenant un coeur solide sur lequel est supporté un principe actif, lesdits granulés étant caractérisés en ce qu'ils comprennent également un composé susceptible de générer un dégagement gazeux constitué d'un agent alcalin.

La présente invention concerne donc des granulés tels que définis dans les revendications.

Les granulés flottants selon l'invention n'utilisent donc pas de mélange agent acide / agent alcalin comme habituellement dans l'état de la technique Dans le cadre de la présente invention, la flottaison est obtenue par réaction du liquide stomacal acide lorsqu'il diffuse au sein de la forme médicamenteuse.

Les granulés flottants selon l'invention sont capables de flotter suffisamment longtemps dans l'estomac et la formulation multicouche assure en outre une diffusion prolongée du principe actif.

Selon la présente invention, l'agent alcalin susmentionné peut être supporté également sur ledit support solide. Ainsi, de tels granulés flottants selon l'invention comprennent, supportés sur ledit coeur solide, au moins un principe actif et un agent alcalin qui est susceptible de générer un dégagement gazeux.

Selon la présente invention, l'agent alcalin susmentionné peut être également utilisé directement comme support. Ainsi, de tels granulés flottants selon l'invention comprennent, supporté sur un coeur solide constitué d'un agent alcalin, au moins un principe actif.

Selon la présente invention, l'agent alcalin est utilisé comme coeur solide sur lequel sont supportés les principes actifs ou il est incorporé dans les couches constitutives du granulé, à savoir déposé sur un support solide avec le ou les principes actifs. Également décrit est un granulé où l'agent alcalin est présent dans une couche déposée par-dessus celle formée par le ou les principes actifs.

Les granulés flottants de l'invention sont donc caractérisés en ce qu'ils ne comprennent pas d'agent acide susceptible de générer un dégagement gazeux. Par "agent acide", on désigne tout acide minéral ou organique, sous forme d'acide libre, d'anhydride d'acide ou de sel d'acide susceptible de générer un dégagement gazeux. Les granulés flottants de l'invention ne comprennent donc pas d'acide carboxylique susceptible de générer un dégagement gazeux (c'est-à-dire pas d'acide mono- ou polycarboxylique).

De préférence, les granulés flottants de l'invention ne comprennent pas d'agent acide en une quantité suffisante pour permettre un dégagement gazeux.

Les granulés flottants de l'invention sont donc de préférence caractérisés en ce qu'ils ne comprennent pas d'acide présentant un pH inférieur ou égal à 4,5 à température ambiante.

De préférence, les granulés flottants de l'invention ne comprennent pas d'acide tartarique, ni d'acide tartrique, ni d'acide citrique, ni d'acide maléique, ni d'acide fumarique, ni d'acide malique, ni d'acide adipique, ni d'acide succinique, ni d'acide lactique, ni d'acide glycolique, ni d'alpha-hydroxy acide, ni d'acide ascorbique, ni d'acides aminés, ni les sels et dérivés de ces acides.

Les granulés flottants selon la présente invention présentent une structure multicouche où le principe actif est déposé sur un support et les autres excipients sont à leur tour déposés autour de ce coeur.

Les granulés flottants selon la présente invention sont caractérisés en ce que l'agent alcalin, utilisé à titre de composé susceptible de générer un dégagement gazeux, n'est pas en contact direct avec un composé acide.

Ainsi, lorsque les granulés flottants selon l'invention comprennent un composé acide, l'agent alcalin et ledit composé acide sont séparés par une couche intermédiaire.

Les granulés flottants selon la présente invention sont des granulés dans lesquels il n'y a aucun contact direct entre un composé à caractère acide et l'agent alcalin utilisé à titre de composé susceptible de générer un dégagement gazeux, ou tout autre composé alcalin. En effet, si les granulés de la présente invention comprennent un composé à caractère acide en tant qu'excipient, ils comprennent alors des films (ou couches) intermédiaires entre ledit acide et ledit agent alcalin pour éviter tout contact entre eux et donc toute réaction entre eux. La présence de tels films intermédiaires permet donc d'obtenir des granulés stables. Ces films (ou couches intermédiaires) jouent le rôle de films protecteurs pour améliorer la stabilité.

Les granulés de l'invention sont donc caractérisés par la présence de couches "barrières" augmentant la stabilité desdits granulés. Ces couches sont utilisées pour empêcher toute réaction entre les différents constituants desdits granulés. Elles permettent ainsi d'éviter l'apparition du phénomène d'effervescence avant la mise en contact des granulés avec le milieu stomacal.

Selon un mode de réalisation des granulés flottants selon la présente invention, l'agent alcalin est déposé sur le support solide : dans un tel cas, les particules de principe actif et de l'agent alcalin sont réparties indifféremment sur le support. Selon un autre mode de réalisation des granulés flottants l'agent alcalin forme une autre couche indépendante de la couche formée par le principe actif.

L'agent alcalin peut donc être incorporé à différents niveaux des granulés. Il peut également être directement utilisé comme support du granulé.

L'expression "granulé" désigne une préparation constituée de grains solides secs, formant chacun un agrégat de particules de poudre d'une solidité suffisante pour permettre diverses manipulations.

Du point de vue physique, les granulés sont des agrégats de particules de poudres diverses cristallisées ou amorphes.

Les granulés de la présente invention sont notamment destinés à une administration par voie orale, et plus particulièrement à être avalés tels quels.

Les granulés de la présente invention présentent une structure caractéristique de type coeur-écorce, le coeur n'étant pas de même nature que les composés formant l'écorce.

Ainsi, ces granulés présentent une structure multicouche. En effet, le principe actif est déposé sur le coeur et donc forme une couche (ou écorce) déposée autour de ce coeur (ou support).

Le coeur des granulés peut également être considéré comme étant un support sur lequel vont se fixer les particules du principe actif.

Le coeur est constitué de particules solides et le principe actif supporté par ledit coeur est également sous forme solide.

La présente invention est donc basée sur la mise au point d'une nouvelle forme orale multiparticulaire.

Les granulés de l'invention présentent une couche de principe actif.

En fonction des paramètres pharmacologiques finaux souhaités, cette première couche peut être recouverte par d'autres couches polymériques utilisant différents polymères d'enrobage ainsi que les différents adjuvants couramment utilisés (plastifiants, solubilisants, lubrifiants, anti-adhérants, etc..). Ces couches peuvent donc comme décrit précédemment jouer le rôle de couches de protection, évitant le contact entre les composés acides et alcalins.

Les granulés flottants de l'invention comprennent un coeur solide choisi de préférence parmi les supports insolubles, et plus particulièrement choisi dans le groupe constitué des polyols, des gommes, des dérivés de la silice, des dérivés de calcium ou de potassium, des composés minéraux tels que les phosphates dicalciques et les phosphates tricalciques, du saccharose, des dérivés de cellulose, notamment de la cellulose microcristalline, de l'éthylcellulose et de l'hydroxypropylméthylcellulose, de l'amidon, des gluconates, des silicates, des cristaux de sucre, et des mélanges de ceux-ci.

Selon un mode de réalisation particulier, comme indiqué ci-dessus, le coeur solide peut être constitué de l'agent alcalin. Une famille particulière de granulés selon l'invention est donc constituée de granulés flottants tels que définis ci-dessus dans lesquels le ou les principes actifs sont déposés sur du bicarbonate de sodium (constituant à la fois le coeur des granulés et l'agent alcalin susceptible de générer un dégagement gazeux).

Le coeur solide des granulés peut également être constitué d'un mélange de composés, notamment d'un mélange de supports insolubles. Ainsi, on peut notamment citer le mélange formé de saccharose et d'amidon ou de composés minéraux dérivés de la silice ou du calcium.

Le coeur solide peut également être constitué de supports solubles parmi lesquels on peut citer certains grades solides de PEG (notamment PEG 4000 ou PEG 6000).

L'expression "dérivés de la silice" désigne la silice ainsi que les silices précipitées obtenues à partir de silicates alcalins, notamment Aerosil^{®}, ou encore le talc, la bentonite ou le kaolin.

L'expression "dérivés de calcium" désigne des excipients cristallins dérivés de l'hydroxyde de calcium, des produits insolubles dans l'eau utilisés en médecine comme diluants, ou des agents de charges et également abrasifs.

L'expression "dérivés de potassium" désigne notamment le bicarbonate de potassium et le chlorure de potassium.

Parmi les supports insolubles formant le coeur des granulés de l'invention, on peut également citer les dérivés du magnésium (notamment carbonates ou oxydes).

De préférence, dans les granulés flottants selon l'invention, l'agent alcalin est choisi dans le groupe constitué des carbonates et bicarbonates, et est notamment choisi dans le groupe constitué du bicarbonate de sodium, du carbonate de sodium, du carbonate de glycine de sodium, du bicarbonate de potassium, du carbonate de magnésium, du carbonate de calcium et de leurs mélanges.

Les granulés de l'invention peuvent également comprendre un liant.

Le rôle du liant est de lier entre elles les particules, c'est-à-dire de parfaire la cohésion du granulé. Ainsi, les liants permettent d'assurer une bonne cohésion du principe actif et du coeur dans les granulés.

Ainsi, les liants se trouvent, comme le principe actif, déposés autour du coeur des granulés.

Comme liants, on peut citer la plupart des excipients hydrophiles qui donnent des solutions visqueuses : gommes arabique et adragante, méthylcellulose et carboxyméthylcellulose, gélatine, amidons, maltodextrines, PEG 4000 et 6000 en solution alcoolique, polyvidone en solution aqueuse ou alcoolique, et aussi des solutions de saccharose, de glucose ou de sorbitol.

Les liants des granulés de l'invention sont de préférence choisis dans le groupe constitué de l'amidon, du saccharose, de la gomme arabique, de la polyvinylpyrrolidone (PVP ou polyvidone), de l'hydroxypropylméthylcellulose (HPMC), de la gomme laque, de l'hydroxypropylcellulose (HPC), de la cellulose, des polyols, des glycérides polyglycolysés (Gelucire^{®}) ou des macrogolglycérides, notamment macrogolglycérides de stéaroyle, également dérivés acryliques, ainsi que des mélanges de ceux-ci.

Parmi les polyols, on peut citer notamment le mannitol, le sorbitol, le maltitol ou le xylitol.

Selon un mode de réalisation particulier, les liants sont de préférence choisis dans le groupe constitué de la polyvinylpyrrolidone, de la gomme laque, des polyols, des glycérides polyglycolysés (Gelucire^{®}) ou des macrogolglycérides, notamment macrogolglycérides de stéaroyle, ainsi que des mélanges de ceux-ci.

On peut également utiliser un liant choisi dans les groupes cités ci-dessus pour des propriétés particulières ; par exemple il peut être utile d'utiliser comme liant des excipients pH-dépendants tels que EUDRAGIT^{®} L100 ou de la gomme laque. On peut également choisir d'utiliser préférentiellement des glycérides polyglycolysés (Gelucire^{®}) pour leur caractère hydrophobe.

Les granulés enrobés sont constitués de grains enrobés d'une ou de plusieurs couches de mélanges d'excipients divers.

Ainsi, les granulés enrobés préférés selon la présente invention comprennent une couche supplémentaire constituée de l'agent d'enrobage.

De préférence, les agents d'enrobage présents dans les granulés de l'invention ne sont pas acides.

Les granulés de l'invention peuvent également comprendre un enrobage constitué par un agent d'enrobage choisi dans le groupe constitué des dérivés de cire, des plastifiants (agents filmogènes), de la gomme laque, de la polyvinylpyrrolidone, du polyéthylène glycol, des dérivés cellulosiques tels que HPMC ou HPC, du saccharose, des glycérides d'acides gras et des polymères méthacryliques.

L'expression "dérivés de cire" désigne des produits naturels ou synthétiques constitués d'esters d'acides gras et d'alcools, en général solides à température ambiante, utilisés à différents titres dans les préparations médicamenteuses.

Les granulés flottants de l'invention peuvent également être enrobés par un film d'enrobage dans lequel sont ajoutés un ou plusieurs excipients tels que des lubrifiants, des colorants, des édulcorants, des plastifiants ou des agents anti-adhérants.

Les granulés de l'invention peuvent aussi comprendre un enrobage entérique, notamment constitué de polymères méthacryliques, notamment Eudragit^{®} L, de gomme laque ou de HPMCP (hydroxypropylméthyl-cellulosephtalate - hypromellose phtalate). De tels granulés sont donc gastro-résistants.

La présence de cet enrobage entérique peut influer sur la biodisponibilité du principe actif, notamment en évitant sa dégradation en milieu acide.

Les granulés flottants de l'invention peuvent également comprendre un enrobage pour libération prolongée.

De tels granulés permettent une libération modifiée ou retardée des principes actifs (granulés à libération modifiée).

Un tel enrobage est obtenu avec des agents d'enrobage notamment constitués de copolymères de méthacrylates et d'acrylates Eudragit^{®} S100, Eudragit^{®} RS, Eudragit^{®} RL, Eudragit^{®} RS, Eudragit^{®} 30D, Eudragit^{®} RL30D, de gomme laque, de dérivés de la cellulose, notamment d'éthylcellulose, de cires (notamment Gelucire^{®}) et de dérivés acryliques.

La présence de cet enrobage pour libération modifiée influence notamment la demi-vie apparente du principe actif.

Les granulés flottants selon la présente invention peuvent également comprendre un lubrifiant et/ou un arôme et/ou un édulcorant et/ou un colorant.

Parmi les lubrifiants mis en oeuvre dans le cadre de la présente invention, on peut notamment citer le talc, le stéarate de magnésium, les dérivés de silice (notamment Aerosil^{®}) ou les cires.

Parmi les arômes mis en oeuvre dans le cadre de la présente invention, on peut citer les arômes classiquement utilisés dans les additifs alimentaires.

Les édulcorants mis en oeuvre dans le cadre de la présente invention sont notamment ceux listés dans la directive 94/35/CE du 30 juin 1994 concernant les édulcorants destinés à être employés dans les denrées alimentaires (modifiée par la directive 2006/25/CE du 5 juillet 2006). Ainsi, on peut notamment citer l'aspartame E 951, le sorbitol E 420, le mannitol E 421, l'acésulfame-K E 950, la saccharine E 954, stevia ou la thaumatine.

Les colorants mis en oeuvre dans le cadre de la présente invention sont notamment ceux listés dans la directive 95/45/CE du 26 juillet 1995 concernant les colorants pouvant être employés dans les denrées alimentaires (modifiée par la directive 2006/33/CE du 20 mars 2006). Ainsi, on peut notamment citer les colorants E 100 à E 180.

Les principes actifs utilisés pour la préparation des granulés flottants de l'invention sont des principes actifs à fenêtre d'absorption étroite ou absorbés haut dans le tube digestif, tels que revendiqués.

De préférence, les principes actifs ne sont pas des composés acides.

Parmi les principes actifs utilisés pour la préparation des granulés flottants selon l'invention, on peut citer notamment : le furosémide, le tiapride, l'alfuzosine, le captopril, le GHB, la metformine, la nifédipine, la buprénorphine, le modafinil, la méthadone, la nalbuphine ou le tétrahydrocannabinol. De préférence, le principe actif n'est pas l'aténolol.

On peut également citer les principes actifs présentant une absorption moins spécifique et de façon non exhaustive les substances antivirales, les agents antidépresseurs, les agents cytostatiques, les agents hypocholestérolémiants, les antalgiques, les analgésiques anti-inflammatoires, les diurétiques et toute autre classe thérapeutique.

La forme galénique décrite ici présente également un intérêt dans les domaines vétérinaires, nutraceutiques, cosmétiques et de l'agriculture.

Parmi les principes actifs, on peut citer les antalgiques et les analgésiques. Les analgésiques permettent d'éliminer la douleur du patient. Parmi les classes d'analgésiques, on peut notamment citer les analgésiques centraux

morphiniques (dérivés de morphine), les analgésiques centraux non morphiniques, les analgésiques périphériques et autres tels que les benzodiazépines.

Le sulfate de morphine, l'oxycodone, l'acide gamma-hydroxybutyrique ou l'un de ses sels, le dextropropoxyphène, le tramadol, et les benzodiazépines sont également cités.

Selon un mode de réalisation préféré, les granulés flottants selon l'invention comprennent de 0,5% à 60%, de préférence de 15% à 50%, en poids de principe actif par rapport au poids total du granulé.

De préférence, les granulés flottants de l'invention comprennent de 15% à 70%, de préférence de 25% à 50%, en poids d'agent alcalin par rapport au poids total du granulé.

De préférence, les granulés flottants de l'invention sont caractérisés en ce que le coeur solide représente de 20% à 80% en poids par rapport au poids total du granulé.

Selon un mode de réalisation particulièrement préféré, les granulés flottants selon l'invention présentent un diamètre inférieur à 3 mm.

Cette taille inférieure à trois millimètres permet de garantir une bonne flottaison dans le liquide stomacal.

En raison du petit diamètre des granulés de l'invention, et a fortiori de leur faible masse, ceux-ci peuvent très facilement flotter dans le liquide stomacal, et ce sans nécessiter la présence d'une quantité trop importante de composé susceptible de générer un dégagement gazeux (agent alcalin). Toutefois, il est connu que des formes pharmaceutiques de petite taille présentent l'inconvénient d'avoir un temps de séjour trop court dans l'estomac. Il est donc inattendu que les granulés de l'invention ne présentent pas cet inconvénient. En effet, ceux-ci présentent un temps de séjour dans l'estomac satisfaisant en raison de leur structure spécifique et des modifications chimiques effectuées (présence des couches 'isolantes' entre les acides et les bases).

Ainsi, les granulés flottants de l'invention peuvent également être dénommés "microgranulés flottants".

La présente invention concerne également un procédé de préparation de granulés flottants tel que défini ci-dessus, caractérisé en ce qu'il comprend une étape d'application par poudrage du principe actif sur un support particulaire solide.

Le procédé comprend également une étape consistant à ajouter l'agent alcalin.

Selon un mode de réalisation particulier, l'agent alcalin est ajouté directement en mélange avec le principe actif et poudré sur le support. Un tel procédé consiste donc à appliquer par poudrage le principe actif et l'agent alcalin sur un support particulaire solide.

Selon un mode de réalisation particulier, l'agent alcalin est utilisé comme support solide. Ainsi, un tel procédé consiste donc à appliquer par poudrage le principe actif sur l'agent alcalin constituant un support particulaire solide.

Le procédé de l'invention peut également comprendre, après l'étape de poudrage, une étape d'enrobage du granulé, notamment en déposant par pelliculage l'agent enrobant sous forme de film sur le granulé, suivie, le cas échéant d'une étape de mélange avec un lubrifiant et/ou un arôme et/ou un édulcorant et/ou un colorant.

La structure des granulés de l'invention est liée à la mise en oeuvre de ce procédé particulier qui permet l'obtention de granulés de structure coeur-écorce.

L'étape de poudrage susmentionnée du procédé pour la préparation des granulés de l'invention peut également comprendre une étape de pulvérisation d'une solution alcoolique ou hydroalcoolique ou aqueuse d'un liant.

Cette étape de pulvérisation et l'étape de poudrage sont de préférence effectuées de façon simultanée ou alternée.

De préférence, l'étape de poudrage susmentionnée est effectuée de façon concomitante avec une étape de pulvérisation d'un liant sous forme de solution.

La combinaison de ces étapes permet d'assurer une bonne cohésion du principe actif sur le coeur des granulés.

Une mise en oeuvre avantageuse du procédé de l'invention consiste ainsi à appliquer le principe actif, mélangé ou non avec l'agent alcalin, sous forme de poudre sur le support particulaire susmentionné (ou coeur des granulés) en alternant des séquences de pulvérisation du liant sous forme de solution.

Le procédé de l'invention peut également comprendre, après l'étape de poudrage, une ou plusieurs étapes d'enrobage du granulé, notamment en déposant par pelliculage le ou les agents enrobants sous forme de films sur le granulé.

Un mode de réalisation préféré du procédé de l'invention consiste en un procédé comprenant, après l'étape d'enrobage, une étape de mélange avec un lubrifiant et/ou un arôme et/ou un édulcorant et/ou un colorant, ceux-ci pouvant être eux-mêmes préparés sous forme de granulés afin d'être finalement mélangés aux granulés actifs.

Toutefois les lubrifiants, arômes, édulcorants et colorants peuvent être également ajoutés avant l'étape de poudrage susmentionnée.

### EXEMPLES

Les exemples ci-après sont cités comme simples références et ne sont pas couverts par les revendications.

**Exemple 1 : Granulés flottants à base de furosémide**

| **Matières Premières Sèches** | mg | % |
|---|---|---|
| Furosémide | 60,000 | 18,13 |
| Neutres taille 550-750 | 100,000 | 30,211 |
| PVP K30 | 20,000 | 6,04 |
| | | |
| Gélucire 50/02 | 30,000 | 9,06 |
| Carbonate de calcium | 30,000 | 9,06 |
| Bicarbonate de Na | 60,000 | 18,13 |
| | | |
| Aquacoat EC30D | 25,000 | 7,55 |
| Dibutylsébaçate | 6,000 | 1,81 |
| | | |
| **Solvants** | | |
| Alcool 96° | Qs | |
| Eau purifiée | Qs | |
| | | |
| *Masse Théorique* | Qs | |
| *Masse Sèche Théorique* | 331,000 | 100,00 |
| *Teneur Théorique (mg*/*g)* | 181,27 | |
| | | |

Les granulés susmentionnés sont obtenus en suivant le mode opératoire suivant :
Dans un premier temps, on a effectué une étape de montage par poudrage du principe actif furosémide sur les neutres supports avec une pulvérisation intermittente d'une solution alcoolique du liant PVP.

On a ensuite effectué un premier enrobage des granulés obtenus précédemment en ajoutant les agents alcalins (carbonate de calcium et bicarbonate de sodium) ainsi qu'un composé de type cire (Gelucire^{®}).

Enfin, on a effectué un deuxième enrobage des granulés avec une suspension aqueuse comprenant un plastifiant (dibutylsébaçate) et l'agent d'enrobage Aquacoat^{®} EC30D.

**Exemple 2 : Granulés flottants à base de nifédipine**

| **Matières Premières Sèches** | mg | % |
|---|---|---|
| Nifédipine | 30,000 | 11,26 |
| Bicarbonate de Na | 150,00 | 56,29 |
| HPMC 603 | 40,00 | 15,01 |
| | | |
| Jaune orangé S | 3,320 | 1,24 |
| | | |
| Eudragit^{®} FS30D | 33,200 | 12,46 |
| Triéthylcitrate | 3,320 | 1,24 |
| Talc | 6,640 | 2,50 |
| | | |
| **Solvants** | | |
| Alcool 96° | Qs | |
| Eau purifiée | Qs | |
| | | |
| *Masse Théorique* | Qs | |
| *Masse Sèche Théorique* | 266,480 | 100,00 |
| *Teneur Théorique (mg*/*g)* | 112,57 | |
| | | |

Les granulés susmentionnés sont obtenus en suivant le mode opératoire suivant :
Dans un premier temps, on a préparé une suspension aqueuse contenant le principe actif (MOR 920) et le liant (HPMC).

Ladite suspension a été ensuite pulvérisée sur le support constitué de bicarbonate de sodium (agent alcalin) et les granulés ont ensuite été séchés dans un lit d'air fluidisé.

On a ensuite effectué un enrobage LP des granulés obtenus précédemment avec une suspension aqueuse comprenant un plastifiant (triéthylcitrate), du talc, l'agent d'enrobage Eudragit^{®} FS30D et le colorant.

**Exemple 3 : Granulés flottants à base de métformine**

| **Matières Premières Sèches** | mg | % |
|---|---|---|
| Métformine | 500,000 | 42,55 |
| Pearlitol 400 DC | 100,00 | 8,51 |
| GLDB | 150,000 | 12,77 |
| | | |
| Ethylcellulose / Eudragit^{®} E100 | 100,000 | 8,51 |
| Bicarbonate de Na | 300,000 | 25,53 |
| Précirol^{®} ATO 5 | 25,000 | 2,13 |
| | | |
| **Solvants** | | |
| Alcool 96° | Qs | |
| | | |
| *Masse Théorique* | Qs | |
| *Masse Sèche Théorique* | 1 175,000 | 100,00 |
| *Teneur Théorique* (*mg*/*g)* | 425,53 | |
| | | |

Les granulés susmentionnés sont obtenus en suivant le mode opératoire suivant :
Dans un premier temps, on a effectué une étape de montage par poudrage du principe actif furosémide sur le support mannitol (Pearlitol 400 DC, Roquette) avec une pulvérisation intermittente d'une solution alcoolique du liant (gomme laque - GLDB).

Les granulés ont ensuite été séchés dans un lit d'air fluidisé.

On a ensuite effectué un enrobage des granulés obtenus précédemment en déposant l'agent alcalin (bicarbonate de sodium) et en pulvérisant une suspension alcoolique comprenant l'agent d'enrobage Eudragit^{®} E100 avec l'éthylcellulose, et finalement le composé Précirol^{®} ATO 5 (Gattefossé).

## Revendications

1. Granulé du type coeur - écorce ayant une taille maximale de 3mm et adapté à flotter à la surface du liquide stomacal comprenant :
a) un coeur neutre solide,
b) une couche interne appliquée sur ledit coeur neutre et contenant un principe actif dont le site d'absorption est situé très haut dans le tube digestif, choisi dans le groupe constitué du furosémide, du tiapride, de l'alfuzosine, du captopril, du GHB, de la metformine, de la nifédipine, de la buprénorphine, du modafinil, de la méthadone, de la nalbuphine, du tétrahydrocannabinol et un agent alcalin susceptible de générer un dégagement gazeux en présence dudit liquide stomacal,
c) une couche externe d'enrobage.

2. Granulé selon la revendication 1, **caractérisé en ce que** ledit agent alcalin est choisi dans le groupe constitué des carbonates et bicarbonates, et est notamment choisi dans le groupe constitué du bicarbonate de sodium, du carbonate de sodium, du carbonate de glycine de sodium, du bicarbonate de potassium, du carbonate de magnésium et du carbonate de calcium.

3. Granulé selon l'une des revendications 1 ou 2, **caractérisé en ce que** ledit coeur solide est choisi parmi les supports insolubles, et plus particulièrement dans le groupe constitué des polyols, les gommes, de la silice, des silices précipitées obtenues à partir de silicates alcalins, du talc, de la bentonite, du kaolin, du carbonate de calcium, du bicarbonate de potassium, du chlorure de potassium, des phosphates dicalciques ou tricalciques, du carbonate de magnésium, de l'oxyde de magnésium, du saccharose, de la cellulose microcristalline, de l'éthyle cellulose ou de l'hydroxypropylméthylcellulose, de l'amidon, des gluconates, des silicates, des cristaux de sucre ou des mélanges de ceux-ci.

4. Granulé selon l'une quelconque des revendications 1 à 3, **caractérisé en ce que** ledit ledit coeur solide supportant ledit principe actif est également constitué dudit agent alcalin.

5. Granulé selon l'une quelconque des revendications 1 à 4, **caractérisé en ce qu'**il comprend en outre un liant choisi dans le groupe constitué des maltodextrines, de l'amidon, du saccharose, des polyols, de la gomme arabique, de la polyvinylpyrrolidone, de la cellulose, de l'hydroxypropylméthylcellulose, de l'hydroxypropylcellulose, de la gomme laque, ou de leurs mélanges.

6. Granulé selon l'une quelconque des revendications 1 à 5, **caractérisé en ce que** ladite couche externe est choisie notamment dans le groupe constitué de la gomme laque, de la polyvinylpyrrolidone, du polyéthylèneglycol, de l'hydroxypropylméthylcellulose, de hydroxypropylcellulose, du saccharose, des glycérides gras, ou de leurs mélanges.

7. Granulé selon l'une quelconque des revendications 1 à 6, **caractérisé en ce qu'**il comprend de 0,5% à 60% en poids de principe actif par rapport au poids total du granulé.

8. Granulé selon l'une quelconque des revendications 1 à 7, **caractérisé en ce qu'**il comprend de 15% à 70% en poids d'agent alcalin par rapport au poids total du granulé.

9. Granulé selon l'une quelconque des revendications 1 à 7, **caractérisé en ce que** le coeur solide représente 20% à 80% en poids par rapport au poids total du granulé.

10. Composition pharmaceutique se présentant sous forme de granulés selon l'une quelconque des revendications 1 à 9.

## Patentansprüche

1. Granulat vom Kern-Rinden-Typ mit einer maximalen Größe von 3 mm und ausgebildet, um auf der Oberfläche der Magenflüssigkeit schwimmfähig zu sein, umfassend:
a) einen festen neutralen Kern,
b) eine auf den neutralen Kern aufgetragene innere Schicht, die ein Wirkstoffprinzip enthält, dessen Absorptionsstelle sich sehr hoch im Verdauungskanal befindet, ausgewählt aus der Gruppe, die aus Furosemid, Tiaprid, Alfuzosin, Captopril, GHB, Metformin, Nifedipin, Buprenorphin, Modafinil, Methadon, Nalbuphin, Tetrahydrocannabinol und einem alkalischen Stoff, der imstande ist, bei Anwesenheit der Magenflüssigkeit eine Gasentwicklung zu erzeugen, besteht
c) eine äußere Hüllschicht.

2. Granulat nach Anspruch 1, **dadurch gekennzeichnet, dass** der alkalische Stoff aus der Gruppe ausgewählt ist, die von den Carbonaten und Bicarbonaten gebildet ist, und vor allem aus der Gruppe ausgewählt ist, die vom Natriumbicarbonat, vom Natriumcarbonat, vom Natriumglycincarbonat, vom Kaliumbicarbonat, vom Magnesiumcarbonat und vom Calciumcarbonat gebildet ist.

3. Granulat nach einem der Ansprüche 1 oder 2, **dadurch gekennzeichnet, dass** der feste Kern aus den unlöslichen Trägern und insbesondere aus der Gruppe ausgewählt ist, die von den Polyolen, den Gummis, der Kieselerde, den ausgefällten Kieselerden, die aus alkalischen Silikaten gewonnnen sind, den Talcum, dem Bentonit, dem Kaolin, dem Calciumcarbonat, dem Kaliumbicarbonat, dem Kaliumchlorid, den Dicalcium- oder Tricalciumphosphaten, dem Magnesiumcarbonat, dem Magnesiumoxid, der Saccharose, der mikrokristallinen Cellulose, der Ethylcellulose oder der Hydroxypropylmethylcellulose, der Stärke, den Gluconaten, den Silikaten, den Zuckerkristallen oder deren Gemischen gebildet ist.

4. Granulat nach einem der Ansprüche 1 bis 3, **dadurch gekennzeichnet, dass** der feste, das Wirkprinzip tragende Kern ebenfalls aus dem alkalischen Stoff gebildet ist.

5. Granulat nach einem der Ansprüche 1 bis 4, **dadurch gekennzeichnet, dass** es ferner ein Bindemittel umfasst, das aus der Gruppe ausgewählt ist, die von den Maltodextrinen, der Stärke, der Saccharose, den Polyolen, dem Gummi arabicum, dem Polyvinylpyrrolidon, der Cellulose, der Hydroxypropylmethylcellulose, der Hydroxypropylcellulose, dem Schellack oder deren Gemischen gebildet ist.

6. Granulat nach einem der Ansprüche 1 bis 5, **dadurch gekennzeichnet, dass** die äußere Schicht vor allem aus der Gruppe ausgewählt ist, die vom Schellack, dem Polyvinylpyrrolidon, dem Polyethylenglycol, der Hydroxypropylmethylcellulose, Hydroxypropylcellulose, der Saccharose, den Fettglyceriden oder deren Gemischen gebildet ist.

7. Granulat nach einem der Ansprüche 1 bis 6, **dadurch gekennzeichnet, dass** es 0,5 bis 60 Gew.-% Wirkstoff in Bezug auf das Gesamtgewicht des Granulats umfasst.

8. Granulat nach einem der Ansprüche 1 bis 7, **dadurch gekennzeichnet, dass** es 15 bis 70 Gew.-% alkalischen Stoff in Bezug auf das Gesamtgewicht des Granulats umfasst.

9. Granulat nach einem der Ansprüche 1 bis 7, **dadurch gekennzeichnet, dass** der feste Kern 20 bis 80 Gew.-% in Bezug auf das Gesamtgewicht des Granulats umfasst.

10. Pharmazeutische Zusammensetzung in Form von Granulaten nach einem der Ansprüche 1 bis 9.

## Claims

1. A granule of core-shell type having a maximum size of 3 mm and adapted to float on the surface of stomach liquid, comprising:
a) a solid neutral core;
b) an inner layer applied to said neutral core and containing an active ingredient having an absorption site high up in the digestive tract, selected from the group formed by furosemide, tiapride, alfuzosine, captopril, GHB, metformin, nifedipine, buprenorphine, modafinil, methadone, nalbuphine, tetrahydrocannabinol and an alkaline agent able to generate gas release in the presence of said stomach liquid;
c) and an outer coating layer.

2. The granule according to claim 1 **characterized in that** said alkaline agent is selected from the group formed by carbonates and bicarbonates, and is particularly selected from the group formed by sodium bicarbonate, sodium carbonate, sodium glycine carbonate, potassium bicarbonate, magnesium carbonate and calcium carbonate.

3. The granule according to either of claims 1 or 2 **characterized in that** said solid core is selected from among insoluble substrates and more particularly from the group formed by polyols, gums, silica, precipitated silicas obtained from alkaline silicates, talc, bentonite, kaolin, calcium carbonate, potassium bicarbonate, potassium chloride, dicalcium or tricalcium phosphates, magnesium carbonate, magnesium oxide, sucrose, microcrystalline cellulose, ethyl cellulose or hydroxypropylmethylcellulose, starch, gluconates, silicates, sugar crystals or mixtures thereof.

4. The granule according to any of claims 1 to 3 **characterized in that** said solid core carrying said active ingredient is also formed of said alkaline agent.

5. The granule according to any of claims 1 to 4 **characterized in that** it further comprises a binder selected from the group formed by maltodextrins, starch, sucrose, polyols, gum arabic, polyvinylpyrrolidone, cellulose, hydroxypropylmethylcellulose, hydroxypropylcellulose, shellac, or the mixtures thereof.

6. The granule according to any of claims 1 to 5 **characterized in that** said outer layer is particularly selected from the group formed by shellac, polyvinylpyrrolidone, polyethyleneglycol, hydroxypropylmethylcellulose, hydroxypropylcellulose, sucrose, fatty glycerides, or the mixtures thereof.

7. The granule according to any of claims 1 to 6 **characterized in that** it comprises 0.5 % to 60 % by weight of active ingredient relative to the total weight of the granule.

8. The granule according to any of claims 1 to 7 **characterized in that** it comprises 15 % to 70 % by weight of alkaline agent relative to the total weight of the granule.

9. The granule according to any of claims 1 to 7 **characterized in that** the solid core represents 20 % to 80 % by weight relative to the total weight of the granule.

10. A pharmaceutical composition in the form of granules according to any of claims 1 to 9.
